(19) Europäisches Patentamt
European Patent Office
Office européen des brevets

(11) Veröffentlichungsnummer: **0 061 110**
**B1**

(12) # EUROPÄISCHE PATENTSCHRIFT

(45) Veröffentlichungstag der Patentschrift: **08.08.84**

(21) Anmeldenummer: **82102052.6**

(22) Anmeldetag: **13.03.82**

(51) Int. Cl.³: **C 07 C 93/14**, C 07 C 149/42, C 07 C 103/38, C 07 C 123/00, C 07 C 121/75, C 07 C 127/19, A 01 N 33/22, A 01 N 37/24, A 01 N 37/34, A 01 N 37/40, A 01 N 37/52 // C07C87/60, C07C79/355

(54) **Neue Nitroaniline.**

(30) Priorität: **20.03.81 DE 3110894**

(43) Veröffentlichungstag der Anmeldung: **29.09.82 Patentblatt 82/39**

(45) Bekanntmachung des Hinweises auf die Patenterteilung: **08.08.84 Patentblatt 84/32**

(84) Benannte Vertragsstaaten: **AT BE CH DE FR GB IT LI LU NL SE**

(56) Entgegenhaltungen: **EP - A - 0 007 482**

**Die Akte enthält technische Angaben, die nach dem Eingang der Anmeldung eingereicht wurden und die nicht in dieser Patentschrift enthalten sind.**

(73) Patentinhaber: **CELAMERCK GmbH & Co. KG, Binger Strasse 173, D-6507 Ingelheim am Rhein (DE)**

(72) Erfinder: **Buck, Wolfgang, Dr., Dipl.-Chem., In der Dörrwies 37, Ingelheim (DE)**
Erfinder: **Sehring, Richard, Dr., Dipl.-Chem., Frankenstrasse 13, Ingelheim (DE)**
Erfinder: **Linden, Gerbert, Dr., Dipl.-Landwirt, Turnierstrasse 44, Ingelheim (DE)**
Erfinder: **Lust, Siegmund, Dr., Dipl.-Biologe, Klappacher Strasse 2f, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 061 110 B1

**Beschreibung**

Die Erfindung betrifft neue Nitroaniline, herbizide Mittel, die diese Verbindungen als Wirkstoffe enthalten, die Herstellung und die Verwendung der neuen Verbindungen zur Beeinflussung des Pflanzenwachstums.

Die erfindungsgemäßen Nitroaniline entsprechen der allgemeinen Formel

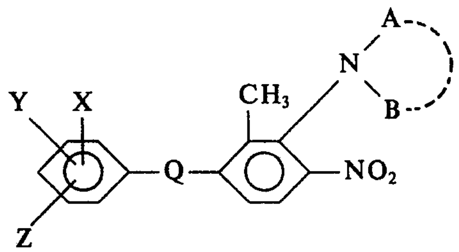

(I)

Darin bedeutet:

A Wasserstoff, Alkyl oder Alkenyl mit bis zu 8 C-Atomen;
B Wasserstoff, Methyl, Acyl oder $CON(CH_3)_2$;
A und B gemeinsam auch einen gegebenenfalls durch Sauerstoff unterbrochenen Alkylenrest mit insgesamt 4 oder 5 Gliedern oder $=CH-N(CH_3)_2$;
Q Sauerstoff oder Schwefel;
X, Y und Z, die gleich oder verschieden sein können, Wasserstoff, Halogen, Methyl, Methoxy, Trifluormethyl, Cyano, COOR oder

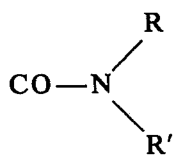

R Wasserstoff, $C_1-C_5$-Alkyl oder Allyl;
R′ Wasserstoff, $C_1-C_5$-Alkyl oder Allyl;
R und R′ gemeinsam auch einen gegebenenfalls durch Sauerstoff unterbrochenen Alkylenrest mit insgesamt 4 oder 5 Gliedern.

Soweit A und B gemeinsam einen Alkylenrest bedeuten, bildet dieser mit dem N-Atom der Gruppe

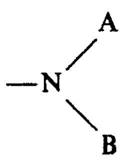

bevorzugt die Reste

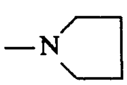 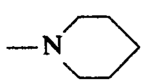 oder 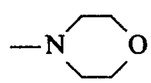

Entsprechendes gilt für die Gruppe

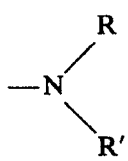

Unter »Halogen« werden hier Fluor, Chlor, Brom und Jod verstanden. Q steht vorzugsweise für Sauerstoff. »Acyl« in der Definition von B steht vor allem für CHO, $COCH_3$, $COC_2H_5$, $COCF_3$, $COC_6H_5$. Die Substituenten X, Y und Z können beliebige Positionen einnehmen. Falls Y und Z Wasserstoff bedeuten, kann X in 2-, 3- oder 4-Position stehen.

Aus der EP-A-0 007 482 sind Nitroaniline bekannt, die im Unterschied zu den Verbindungen der vorliegenden Anmeldung in 2-Stellung der Anilinogruppe ein Chloratom als Substituenten enthalten.

Die bekannten Verbindungen haben sich jedoch im direkten Vergleich im Gewächshausversuch sowohl gegen Ungräser als auch gegen Unkräuter als den erfindungsgemäßen Verbindungen unterlegen erwiesen.

Zur Herstellung der neuen Verbindungen eignen sich folgende Verfahren:

1. Umsetzung eines entsprechenden 3-Chlor-2-methyl-6-nitriloanilins II

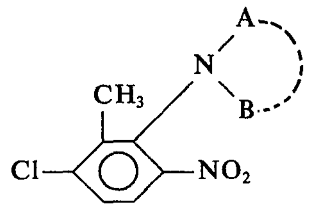

(II)

mit einem Phenol oder Thiophenol III

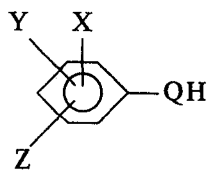

(III)

Die Reaktion erfolgt in Gegenwart eines säurebindenden Mittels, oder man bringt ein Phenolat bzw. Thiophenolat zur Reaktion. Das Phenolat bzw. Thiophenolat, vorzugsweise eine entsprechende Alkaliverbindung, wird zweckmäßig in situ hergestellt. Dazu kann dem Reaktionsgemisch z. B. Kaliumcarbonat zugesetzt werden. Die Umsetzung wird zwischen etwa 20 und 180° C, vorzugsweise oberhalb 50° C, durchgeführt. Dabei kann ein polares organisches Lösungsmittel (beispielsweise Acetonitril, Dimethylformamid, Dimethylsulfoxid, N-Methylpyrrolidon) als Reaktionsmedium dienen. Die Reaktionskomponenten können jedoch auch in der Schmelze umgesetzt werden.

2. Umsetzung eines entsprechenden 3-Methyl-2,4-diphenoxynitrobenzols IV

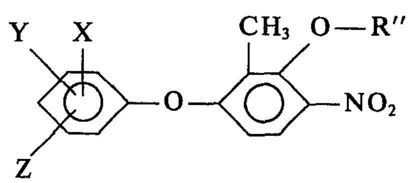

(IV)

mit einem Amin V

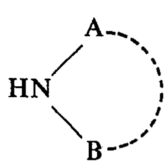

(V)

R″ steht bevorzugt für

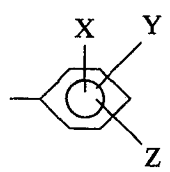

so daß die 2- und die 4-Phenoxygruppe der Verbindung IV gleich sind. Die 2ständige Phenoxygruppe, die während der Reaktion entfernt wird, kann jedoch auch unsubstituiert bzw. anders als die 4ständige Gruppe substituiert sein.

In dem Amin V bedeutet A bevorzugt Alkyl oder Alkenyl, B bevorzugt Wasserstoff oder Methyl. A und B zusammen können auch für einen gegebenenfalls durch Sauerstoff unterbrochenen Alkylenrest mit insgesamt 4 oder 5 Gliedern stehen.

Die Umsetzung erfolgt zwischen etwa 20 und 160° C. Als Reaktionsmedium können inerte organische Lösungsmittel, beispielsweise Dioxan, Tetrahydrofuran, Benzol, Toluol, Xylol, chlorierte Kohlenwasserstoffe, Dimethylsulfoxid, aber auch Wasser Verwendung finden. Die Auswahl des Reaktionsmediums ist nicht kritisch, jedoch wird bevorzugt ein Medium benutzt, in dem die Reaktionskomponenten eine ausreichende Löslichkeit besitzen.

Verbindungen der Formel I, in denen B einen Acylrest bedeutet, können aus den nach 1. oder 2. erhaltenen entsprechenden Verbindungen mit B gleich Wasserstoff durch Acylierung erhalten werden.

So läßt sich die Formylgruppe z. B. mit Ameisensäure/Acetanhydrid einführen, die Trifluoracetylgruppe mit Trifluoressigsäureanhydrid. In anderen Fällen gelingt die Acylierung mit dem entsprechenden Säurechlorid, vorzugsweise in Gegenwart eines Katalysators wie $AlCl_3$.

Verbindungen der Formel I, in denen

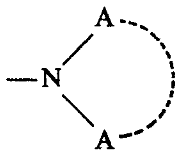

für $-N=CH-N(CH_3)_2$ steht, können gegebenenfalls aus den nach 1. oder 2. erhaltenen Verbindungen mit A und B gleich Wasserstoff durch Umsetzung mit einem Dimethylformamid-Acetal, vorzugsweise einem Diniederalkylacetal, in Gegenwart eines sauren Katalysators, z. B. p-Toluolsulfonsäure, in der Wärme oder durch Umsetzung mit Dimethylformamid/Phosphoroxychlorid gewonnen werden.

Die Ausgangsstoffe sind zum Teil bekannt. Anderenfalls können sie nach üblichen Methoden gewonnen werden.

Hydroxybenzoesäureamide (II, X gleich CONRR') sind über die entsprechenden Hydroxybenzoesäurechloride zugänglich. 3-Chlor-2-methyl-6-nitro-aniline können durch Umsetzung des 2,6-Dichlor-3-nitrotoluols mit Ammoniak oder einem Amin in einem inerten Lösungsmittel, z. B. Dimethylsulfoxid, erhalten werden, bevorzugt bei erhöhter Temperatur.

Verbindungen II mit B gleich Acyl erhält man durch Nitrieren des entsprechenden 3-Chlor-2-methyl-N-acylanilids oder aus Verbindungen II mit B gleich Wasserstoff durch Acylieren.

Die neuen Verbindungen haben herbizide Eigenschaften, sind jedoch auch als Zwischenprodukte von Interesse, da sie aufgrund der vorhandenen Substituenten (z. B. Aminogruppe, Nitrogruppe) vielfältig abgewandelt werden können, etwa zu anderen Pflanzenschutzmitteln oder Arzneistoffen.

Für die Anwendung als Herbizide werden die Verbindungen der Formel I in an sich bekannter Weise mit üblichen Hilfs- und/oder Trägerstoffen zu gebräuchlichen Formulierungen verarbeitet, z. B. zu Emulsionskonzentraten oder Suspensionspulvern, bei denen der Wirkstoffgehalt zwischen etwa 10 und 95 Gewichtsprozent liegt und die für die Ausbringung mit Wasser auf die gewünschte Wirkstoffkonzentration eingestellt werden. Jedoch können auch Präparate hergestellt werden, die unverdünnt angewendet werden, beispielsweise Stäube oder Granulate. Hier liegt der Wirkstoffgehalt zwischen 0,2 und 20 Gewichtsprozent, vorzugsweise zwischen 0,5 und 3 Gewichtsprozent.

## Formulierungsbeispiele

### 1. Suspensionspulver

Zusammensetzung:

| | |
|---|---|
| 25 Gew.-% | einer Verbindung der Formel I |
| 55 Gew.-% | Kaolin |
| 10 Gew.-% | kolloidale Kieselsäure |
| 9 Gew.-% | Calciumligninsulfonat |
| 1 Gew.-% | Natriumtetrapropylenbenzolsulfonat |

### 2. Emulsionskonzentrat

| | |
|---|---|
| 20 Gew.-% | einer Verbindung der Formel I |
| 70 Gew.-% | flüssiges Lösungsmittelgemisch aus hochsiedenden aromatischen Kohlenwasserstoffen (Shellsol A) |
| 6,5 Gew.-% | Tensiofix AS (Emulgator) |
| 3,5 Gew.-% | Tensiofix DS (Emulgator) |

Aus den Konzentraten nach Beispiel 1 und 2 werden durch Vermischen mit Wasser Spritzbrühen hergestellt, die im allgemeinen etwa 0,05 bis 0,5 Gewichtsprozent Wirkstoff enthalten.

3. Stäubemittel

| | |
|---|---|
| 1 Gew.-% | einer Verbindung der Formel I |
| 98 Gew.-% | Talkum |
| 1 Gew.-% | Methylcellulose |

4. Suspensionspulver

| | |
|---|---|
| 95 Gew.-% | einer Verbindung der Formel I |
| 4 Gew.-% | Calciumligninsulfonat |
| 1 Gew.-% | Natriumtetrapropylenbenzolsulfonat |

Es ist auch möglich und in einigen Fällen vorteilhaft, die erfindungsgemäßen Wirkstoffe zusammen mit anderen Herbiziden einzusetzen, z. B. mit Triazin-Herbiziden wie Simazin oder Atrazin in Mais, mit Harnstoff-Herbiziden wie z. B. Linuron oder Monolinuron in Kartoffeln oder Getreide, mit Dinitroanilin-Herbiziden wie Dinitramin oder Trifluralin in Getreide, mit Diphenyläthern, z. B. Nitrophen in Reis, mit Carbonsäureamiden wie Alachlor in Zwiebeln.

Die neuen Verbindungen der Formel I zeigen eine starke Herbizidwirkung sowohl bei Vorauflauf- als auch bei Nachauflaufeinsatz. Sie können zur Bekämpfung verbreiteter Unkräuter und Ungräser wie Ackersenf, Amaranth, Gänsefuß, Kamille, Hühnerhirse, Flughafer, Ackerfuchsschwanz, eingesetzt werden. Aufgrund ihrer selektiven Wirkung können die erfindungsgemäßen Wirkstoffe in zahlreichen Kulturen angewendet werden, wie z. B. in Kartoffeln, Tomaten, Weizen, Mais, Reis, Gerste, Ackerbohnen, Sonnenblumen, Erbsen.

In der nachstehenden Tabelle sind Ergebnisse von Gewächshausversuchen für eine repräsentative Auswahl erfindungsgemäßer Verbindungen bei wichtigen Unkräutern und Ungräsern aufgeführt.

Angegeben ist die Aufwandmenge in kg/ha.

A: Sinapis alba (Weißer Senf, withe mustard)
B: Galium aparine (Klettenlabkraut, catchweed bedstraw)
C: Alopecurus myosuroides (Ackerfuchsschwanz, blackgrass)
D: Echinochloa crus-galli (Hühnerhirse, barnyardgrass)

VA bedeutet Anwendung im Vorauflauf-, NA im Nachauflaufverfahren.

Tabelle

| Verbindung | Anwendungs-zeit | Testpflanzen A | B | C | D |
|---|---|---|---|---|---|
| Beispiel 3 | NA | 1,25 | 0,25 | 1,25 | 1,25 |
| Beispiel 6 | NA | 0,25 | 0,25 | 1,25 | 1,25 |
| Beispiel 13 | VA | 1,25 | 1,25 | 0,25 | <0,25 |
| | NA | 0,25 | <0,25 | 1,25 | 1,25 |
| Beispiel 15 | NA | <0,25 | <0,25 | 1,25 | 1,25 |
| Beispiel 16 | NA | <0,25 | <0,25 | 1,25 | <0,25 |
| Beispiel 50 | VA | <0,25 | >1,0 | 1,0 | 1,0 |
| Beispiel 64 | VA | <0,25 | 1,0 | 1,0 | 1,0 |

Die angegebenen Zahlen sind die $ED_{90}$-Werte (90% Kontrolle der Unkräuter; Bonitierung nach 3 Wochen).

Die folgenden Synthesebeispiele sollen die erfindungsgemäßen Herstellungsverfahren verdeutlichen:

Beispiel 1

2-Methyl-3-phenoxy-6-nitroanilin

a) 3-Chlor-2-methyl-6-nitroanilin

41,2 g (0,2 Mol) 2,6-Dichlor-3-nitrotoluol werden in 200 ml Dimethylsulfoxyd gelöst und in einem 0,5-Ltr.-Schüttelautoklav gegeben. 30 ml flüssiges $NH_3$ werden zugesetzt, der Autoklav wird verschlossen und bei 100°C 24 Stunden geschüttelt, wobei sich ein Maximaldruck von 12 bar einstellt. Nach Abkühlen und Belüften des Autoklavs wird der Ansatz entnommen und auf 1,5 Ltr. Wasser gegossen. Das Produkt fällt aus und wird abgesaugt. Man erhält 34,7 g Rohprodukt (93% d. Th.) als gelben kristallinen Feststoff (Fp. 120—136°C), der durch Umkristallisieren aus Ethanol gereinigt wird. Fp. 144—147°C, Ausbeute 24 g (64% d. Th.).

b) 2-Methyl-3-phenoxy-6-nitroanilin

24 g 3-Chlor-2-methyl-6-nitroanilin (0,12 Mol) und 24 g Phenol (0,255 Mol) werden bei ca. 110°C zusammengeschmolzen und mit 28 g gemahlenem Kaliumcarbonat (0,2 Mol) verrührt. Der Ansatz wird auf 150—160°C erhitzt und bis zur Beendigung der Reaktion bei dieser Temperatur gehalten (ca. 24 Stunden). Die Kontrolle erfolgt dünnschichtchromatographisch. Der Ansatz wird in Toluol gelöst, die Lösung mit 2 N Natronlauge ausgeschüttelt, getrocknet, mit Tonsil behandelt und eingeengt. Man erhält ein gelbbraunes Öl, welches langsam erstarrt; Fp. 73—77°C, Ausbeute 26 g (84% d. Th.). Durch Umkristallisieren aus Ethanol erhält man einen gelben, kristallinen Feststoff, Fp. 76—78°C; Elementaranalyse und NMR-Spektrum bestätigen die erwartete Struktur.

Beispiel 2

2-Methyl-3-phenylthio-6-nitro-anilin

In eine Lösung von 5,6 g 3-Chlor-2-methyl-6-nitroanilin (0,03 Mol) und 3,3 g Thiophenol (0,03 Mol) in 30 ml DMSO werden unter Rühren und Erhitzen 4,2 g gemahlenes Kaliumcarbonat eingetragen. Der Ansatz wird 3 Stunden bei 110°C gerührt, nach Abkühlen mit Wasser verdünnt. Dabei fällt das Produkt aus. Es wird abgesaugt und aus Isopropanol umkristallisiert. Man erhält 7,2 g (92% d. Th.) eines orangefarbenen, kristallinen Feststoffes, Fp. 107—110°C.

Beispiel 3

2-Methyl-3-phenoxy-6-nitro-N-allylanilin

a) 3-Methyl-2,4-diphenoxy-nitrobenzol

12,5 g Natriumphenolat und 10,3 g (0,05 Mol) 2,6-Dichlor-3-nitrotoluol werden in 100 ml DMSO eine Stunde bei 100°C gerührt. Die Lösung wird abgekühlt, mit Salzsäure schwach sauer gestellt und mit Eiswasser verrührt. Das Produkt fällt als Öl aus. Es wird in Methylenchlorid gelöst, mit Wasser ausgeschüttelt, getrocknet und eingeengt. Man erhält 15 g braunes Öl (93% d. Th.), welches durch Säulenchromatographie gereinigt wird (Kieselgel, Toluol). Das Produkt ist ein gelbes Öl (12 g, entspr. 75% d. Th.), das erst bei längerem Stehen fest wird. Fp. 56—57°C (aus Isopropanol).

b) 2-Methyl-3-phenoxy-6-nitro-N-allylanilin

5 g 3-Methyl-2,4-diphenoxy-nitrobenzol (0,0156 Mol) werden in 30 ml DMSO gelöst und mit 5 g Allylamin versetzt. Der Ansatz wird 3 Stunden auf 110°C erhitzt, abgekühlt und mit Wasser verdünnt. Dabei fällt das Produkt als Öl aus. Es wird in Methylenchlorid aufgenommen, mit verd. Natronlauge und Wasser geschüttelt, getrocknet, eingeengt. Der ölige Rückstand wird chromatographisch gereinigt (Kieselgel, Toluol). Man erhält 4 g gelbes Öl (90% d. Th.), RF 0,448 [DC-Fertigplatten Kieselgel 60 $F_{254}$ (Merck), Laufmittel Toluol, 24°C].

## Beispiel 4

### 2-Methyl-3-phenoxy-6-nitro-formanilid

7,32 g 2-Methyl-3-phenoxy-6-nitro-anilin (0,03 Mol) werden in 40 ml Ameisensäure und 3 g Acetanhydrid bei 80°C 2 Stunden gerührt. Während weiterer 10 Stunden bei 80°C werden im 2-Stundenabstand je 1,5 g Acetanhydrid nachgegeben. Der Ansatz wird eingeengt, der Rückstand mit Äther verrührt und abgesaugt. Man erhält 6 g gelbes, kristallines Produkt, Fp. 175—177°C.

## Beispiel 5

### 1,1-Dimethyl-3-(2-methyl-6-nitro-3-phenoxyphenyl)-formamidin

4,88 g 2-Methyl-6-nitro-3-phenoxyanilin (0,02 Mol) werden mit 10 ml Dimethylformamiddimethylacetal und einer Spatelspitze p-Toluolsulfonsäure auf 110°C erhitzt, wobei Methanol abdestilliert wird. Nach 30 Minuten wird der Ansatz eingeengt, der Rückstand in Chloroform gelöst und mit Wasser mehrfach geschüttelt. Beim Einengen der Chloroformlösung erhält man das genannte Produkt als rohes, viskoses Öl. RF: 0,056 Toluol [DC-Fertigplatten Kieselgel 60 $F_{254}$ (Merck), 24°C].

**Entsprechend den vorstehenden Beispielen werden auch folgende Verbindungen der Formel I erhalten:**

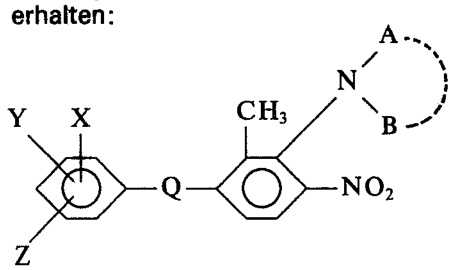

(I)

| Beispiel | A | B | Q | X | Y | Z | Fp. [°C] | RF |
|---|---|---|---|---|---|---|---|---|
| 6 | H | H | O | 4-Cl | H | H | 95–97 | |
| 7 | H | H | O | 4-F | H | H | 93–95 | |
| 8 | H | H | S | 4-Cl | H | H | 126–128 | |
| 9 | H | H | O | 3-Cl | 5-Cl | H | 122–123 | |
| 10 | H | H | O | 2-$Cl_2$ | 5-Cl | H | 88–90 | |
| 11 | H | H | O | 4-$CH_3$ | H | H | 88–90 | |
| 12 | H | H | O | 4-$CH_3O$ | H | H | 101–104 | |
| 13 | n-$C_3H_7$ | H | O | H | H | H | gelbes Öl | 0,455 |
| 14 | H | H | O | 2-Cl | H | H | gelbes Öl | 0,262 |
| 15 | H | H | O | 3-Cl | H | H | 93–95 | |
| 16 | H | H | O | 2-F | H | H | 96–98 | |
| 17 | H | H | O | 2-$CONHC_2H_5$ | H | H | 148–150 | |
| 18 | H | H | O | 2-CN | H | H | 150–154 | |
| 19 | H | H | O | 2-$CH_3$ | H | H | braunes Öl | |
| 20 | H | H | O | 3-$CH_3$ | H | H | 84–88 | |
| 21 | H | H | O | 3-$CONHiC_3H_7$ | H | H | 203–205 | |

Fortsetzung

| Beispiel | A | B | Q | X | Y | Z | Fp. [°C] | RF |
|---|---|---|---|---|---|---|---|---|
| 22 | H | H | O | 3-CON 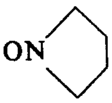 | H | H | 125–128 | |
| 23 | H | H | O | 3-$CONHC_3H_7$ | H | H | 130–134 | |
| 24 | H | H | O | 3-$CON(CH_3)_2$ | H | H | 143–145 | |
| 25 | H | $COCH_3$ | O | H | H | H | 189–191 | |
| 26 | H | $COCF_3$ | O | H | H | H | 116–118 | |
| 27 | H | H | O | 3-$CF_3$ | H | H | 81–83 | |
| 28 | H | H | O | 3-$CONHCH_3$ | H | H | 119–122 | |
| 29 | H | H | O | 3-$CONHCH–C_2H_5$<br>$CH_3$ | H | H | 159–161 | |
| 30 | H | H | O | 4-CON 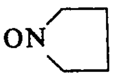 | H | H | 207–211 | |
| 31 | H | $CON(CH_3)_2$ | O | H | H | H | 143–145 | |
| 32 | H | $COC_2H_5$ | O | H | H | H | 149–151 | |
| 33 | H | $COC_6H_5$ | O | H | H | H | 152–154 | |
| 34 | $C_2H_5$ | H | O | H | H | H | | 0,392 |
| 35 | H | H | O | 4-CN | H | H | 213–215 | |
| 36 | $CH_3$ | H | O | H | H | H | gelbes Öl | 0,352 |
| 37 | n-$C_3H_7$ | H | O | 4-$CH_3$ | H | H | Öl | 0,456 |
| 38 | n-$C_3H_7$ | H | S | H | H | H | Öl | 0,48 |
| 39 | H | H | O | 3-CONH<br>$CH_2–CH=CH_2$ | H | H | 128–133 | |
| 40 | H | H | O | 3-CON 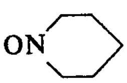 | H | H | 146–148 | |
| 41 | H | H | O | 3-CON 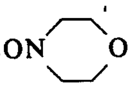 | H | H | 154–156 | |
| 42 | $CH_3$ | $CH_3$ | O | H | H | H | | 0,301 (Toluol/Heptan 1 : 1, 24°C) |
| 43 | $CH(CH_3)_2$ | H | O | H | H | H | | 0,394 |
| 44 | n-$C_4H_9$ | H | O | H | H | H | | 0,503 |
| 45 | $CH_3$ | CHO | O | H | H | H | 92–93 | |

Fortsetzung

| Beispiel | A | B | Q | X | Y | Z | Fp. [°C] | RF |
|---|---|---|---|---|---|---|---|---|
| 46 | $CH_2CH(CH_3)_2$ | H | O | H | H | H | | 0,480 (Toluol/ Heptan 1 : 1, 23°C) |
| 47 | H | H | O | 4-Br | H | H | 79–81 | |
| 48 | H | H | O | 4-J | H | H | | 0,215 |
| 49 | H | H | O | 2-Cl | 4-Br | 5-Cl | | |
| 50 | $C_2H_5$ | H | O | H | H | H | | 0,392 |
| 51 | $C_2H_5$ | $COCF_3$ | O | H | H | H | | 0,236 |
| 52 | $C_2H_5$ | H | O | 4-F | H | H | | 0,428 |
| 53 | $CH_3$ | H | S | H | H | H | Öl | 0,365 |
| 54 | n-$C_3H_7$ | H | O | 4-F | H | H | Öl | 0,461 |
| 55 | $C_2H_5$ | $CH_3$ | O | 2-$CH_3$ | 4-$CH_3$ | H | | |
| 56 | H | H | H | 2-$OCH_3$ | H | H | 100–102 | |
| 57 | $-CH_2-CH=CH_2$ | H | O | 3-$CH_3$ | 5-$CH_3$ | H | | 0,441 |
| 58 | $CH_3$ | H | O | 4-$CH_3$ | H | H | Öl | 0,3438 |
| 59 | $CH(CH_3)_2$ | H | O | 4-F | H | H | | 0,407 |
| 60 | n-$C_4H_9$ | H | O | 4-F | H | H | | 0,524 |
| 61 | n-$C_4H_9$ | H | S | H | H | H | | 0,531 |
| 62 | n-$C_4H_9$ | H | O | 4-$CH_3$ | H | H | | 0,514 |
| 63 | $CH_3$ | H | S | H | H | H | | 0,365 |
| 64 | $CH_3$ | H | O | 4-F | H | H | 60–63 | |
| 65 | $CH_3$ | H | O | 4-$CH_3$ | H | H | | 0,344 |
| 66 | n-$C_4H_9$ | H | S | H | H | H | | 0,531 |
| 67 | n-$C_4H_9$ | H | O | 4-$CH_3$ | H | H | | 0,514 |
| 68 | $CH(CH_3)_2$ | H | S | H | H | H | | 0,426 |
| 69 | $CH(CH_3)_2$ | H | O | 4-$CH_3$ | H | H | | 0,440 |
| 70 | $C_2H_5$ | H | S | H | H | H | 65–68 | |
| 71 | $C_2H_5$ | H | O | 4-$CH_3$ | H | H | | 0,432 |
| 72 | $C_2H_5$ | H | O | 2-$CH_3$ | 4-$CH_3$ | H | | 0,357 |

Anmerkung zu den Tabellen:
RF-Werte: auf DC-Fertigplatten Kieselgel 60 $F_{254}$ (Merck/Darmstadt);
Laufmittel Toluol, Temperatur 24°C (sofern nichts anderes vermerkt ist).

**Patentansprüche**

1. Verbindungen der Formel

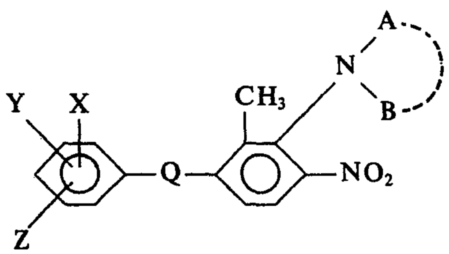

(I)

in der

A Wasserstoff, Alkyl oder Alkenyl mit bis zu 8 C-Atomen;
B Wasserstoff, Methyl, Acyl oder $CON(CH_3)_2$;
A und B gemeinsam auch einen gegebenenfalls durch Sauerstoff unterbrochenen Alkylenrest mit insgesamt 4 oder 5 Gliedern oder $=CH-N(CH_3)_2$;
Q Sauerstoff oder Schwefel;
X, Y und Z, die gleich oder verschieden sein können, Wasserstoff, Halogen, Methyl, Methoxy, Trifluormethyl, Dyano, COOR oder

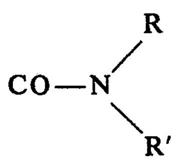

R Wasserstoff, $C_1-C_5$-Alkyl oder Allyl;
R′ Wasserstoff, $C_1-C_5$-Alkyl oder Allyl;
R und R′ gemeinsam auch einen gegebenenfalls durch Sauerstoff unterbrochenen Alkylenrest mit insgesamt 4 oder 5 Gliedern bedeuten.

2. 2-Methyl-3-phenoxy-6-nitro-N-ethylanilin
3. 2-Methyl-3-(4-chlorphenoxy-6-nitro-anilin
4. 2-Methyl-3-(4-fluorphenoxy)-6-nitro-anilin
5. Herbizide Mittel, gekennzeichnet durch einen Gehalt an einer Verbindung nach Anspruch 1 bis 4.
6. Verwendung von Verbindungen nach Anspruch 1 bis 4 zur Bekämpfung unerwünschten Pflanzenwuchses.
7. Verwendung von Verbindungen nach Anspruch 1 bis 4 zur selektiven Bekämpfung von Unkräutern und Ungräsern in Nutzpflanzenkulturen.
8. Verwendung von Verbindungen nach Anspruch 1 bis 4 zur selektiven Unkraut- und Ungrasbekämpfung in Mais, Weizen, Gerste, Reis, Kartoffeln, Erbsen, Ackerbohnen, Tomaten, Sonnenblumen.
9. Verfahren zur Herstellung von Verbindungen nach Anspruch 1 bis 4, dadurch gekennzeichnet, daß man

a) ein entsprechendes 3-Chlor-2-methyl-6-nitroanilin II

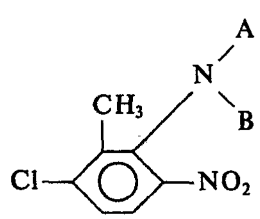

(II)

mit einem Phenol bzw. Thiophenol III

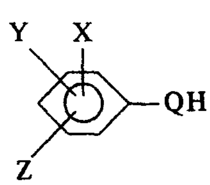

(III)

in Form des Phenolats bzw. Thiophenolats oder in Gegenwart eines säurebindenden Mittels umsetzt
oder daß man
b) ein entsprechendes 3-Methyl-2,4-diphenoxy-nitrobenzol IV

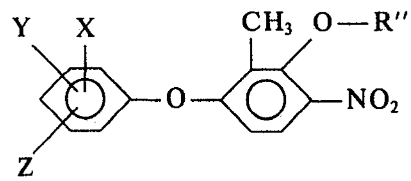

(IV)

in der R'' für einen gegebenenfalls substituierten Phenylrest, vorzugsweise den Rest

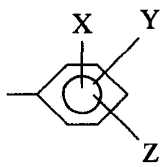

steht, mit einem Amin V,

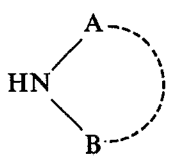

(V)

umsetzt

und daß man gegebenenfalls zur Herstellung solcher Verbindungen der Formel I, in denen B eine Acylgruppe bedeutet, in eine entsprechende Verbindung mit B gleich Wasserstoff nach üblichen Methoden eine Acylgruppe einführt oder daß man gegebenenfalls zur Herstellung solcher Verbindungen der Formel I, in denen

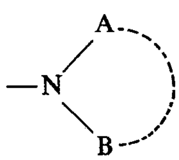

für $-N=CH-N(CH_3)_2$ steht, eine entsprechende Verbindung mit A und B gleich Wasserstoff mit einem Dimethylformamid-Acetal oder mit Dimethylformamid/Phosphoroxychlorid umsetzt.

10. Verfahren zur Herstellung neuer herbizider Mittel, dadurch gekennzeichnet, daß man eine Verbindung nach Anspruch 1 bis 4 mit üblichen Hilfs- und/oder Trägerstoffen zu gebräuchlichen herbiziden Konzentraten bzw. anwendungsfertigen Mitteln verarbeitet.

**Claims**

1. Compounds of formula

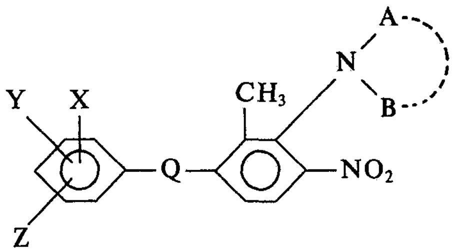

(I)

wherein

A represents hydrogen, alkyl or alkenyl with up to 8 carbon atoms;
B represents hydrogen, methyl, acyl or $CON(CH_3)_2$;
A and B together may also represent an alkylene group with a total of 4 or 5 members, optionally interrupted by oxygen, or $=CH-N(CH_3)_2$;
Q represents oxygen or sulphur;
X, Y and Z, which may be the same or different, represent hydrogen, halogen, methyl, methoxy, trifluoromethyl, cyano, COOR or

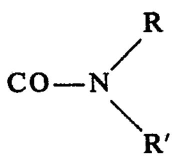

R represents hydrogen, $C_{1-5}$ alkyl or allyl;
R′ represents hydrogen, $C_{1-5}$ alkyl or allyl;
R and R′ together may also represent an alkylene group with a total of 4 or 5 members optionally interrupted by oxygen.

2. 2-Methyl-3-phenoxy-6-nitro-N-ethylaniline.
3. 2-Methyl-3-(4-chlorophenoxy-6-nitro-aniline.
4. 2-Methyl-3-(4-fluorophenoxy)-6-nitro-aniline.
5. Herbicidal agents, characterised in that they contain a compound as claimed in claims 1 to 4.
6. Use of compounds as claimed in claims 1 to 4 for combating undesirable plant growth.
7. Use of compounds as claimed in claims 1 to 4 for selectively combating weeds and wild grasses in cultivated crops.
8. Use of compounds as claimed in claims 1 to 4 for selectively combating weeds and wild grasses in maize, wheat, barley, rice, potatoes, peas, broad beans, tomatoes and sunflowers.
9. Process for the preparation of compounds as claimed in claims 1 to 4, characterised in that

a) a corresponding 3-chloro-2-methyl-6-nitroaniline II

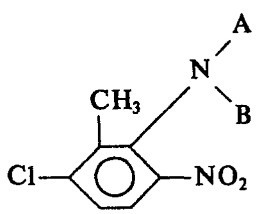

(II)

is reacted with a phenol or thiophenol III

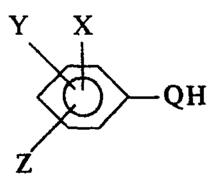

(III)

in the form of the phenoxide or thiophenoxide or in the presence of an acid-binding agent, or

b) a corresponding 3-methyl-2,4-diphenoxynitrobenzene IV

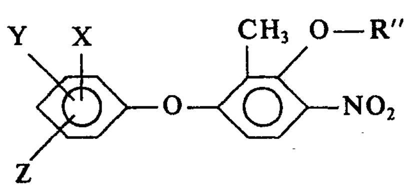

(IV)

wherein R″ represents an optionally substituted phenyl group, preferably the group

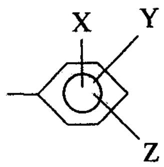

is reacted with an amine V

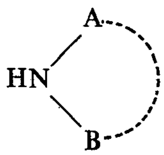

(V)

and if desired, in order to prepare compounds of formula I wherein B represents an acyl group, an acyl group is introduced into a corresponding compound wherein B is hydrogen, by conventional methods, or if desired, in order to prepare compounds of formula I wherein

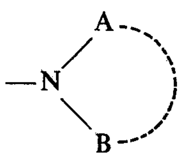

represents $-N=CH-N(CH_3)_2$ a corresponding compound wherein A and B represent hydrogen is reacted with a dimethylformamide-acetal or with dimethylformamide/phosphorus oxychloride.

10. Process for the preparation of new herbicidal agents, characterised in that a compound as claimed in claims 1 to 4 is processed with conventional excipients and/or carriers to form conventional herbicidal concentrates or compositions ready for use.

**Revendications**

1. Composés de formule

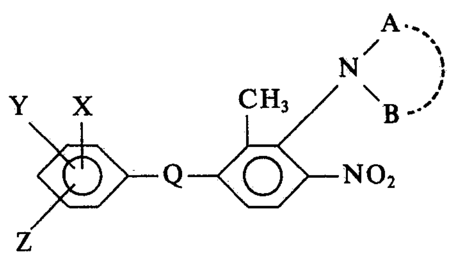

(I)

dans laquelle:

A représente hydrogène, alcoyle ou alcényle avec jusqu'à 8 atomes de C;

B représente hydrogène, méthyle, acyle ou $CON(CH_3)_2$;
A et B représentent également ensemble un radical alcoylène avec en tout 4 ou 5 membres, éventuellement interrompu par oxygène ou $=CH-N(CH_3)_2$;

Q représente oxygène ou soufre;

X,Y et Z, qui peuvent être identiques ou différents, représentent hydrogène, halogène, méthyle, méthoxy, trifluorométhyle, cyano, COOR ou

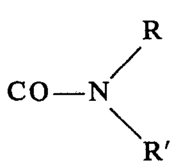

R représente hydrogène, alcoyle en $C_1-C_5$ ou allyle;
R' représente hydrogène, alcoyle en $C_1-C_5$ ou allyle;
R et R' représentent également ensemble un radical alcoylène avec en tout 4 ou 5 membres, éventuellement interrompu par oxygène.

2. La 2-méthyl-3-phénoxy-6-nitro-N-éthylaniline.
3. La 2-méthyl-3-(4-chlorophénoxy)-6-nitroaniline.
4. La 2-méthyl-3-(4-fluorophénoxy)-6-nitroaniline.
5. Agent herbicide, caractérisé par une teneur en un composé selon la revendication 1à 4.
6. Utilisation de composés selon la revendication 1 à 4, pour lutter contre la croissance indésirable de plantes.
7. Utilisation de composés selon la revendication 1 à 4, pour la lutte sélective contre les plantes indésirables et mauvaises herbes dans les cultures de plantes utiles.
8. Utilisation de composés selon la revendication 1 à 4, pour la lutte sélective contre les plantes indésirables et mauvaises herbes dans le maïs, le froment, les oignons, le riz, les pommes de terre, les petits pois les haricots des champs, les tomates, les tournesols.
9. Procédé pour la préparation de composés selon la revendication 1 à 4, caractérisé en ce que

a) on fait réagir une 3-chloro-2-méthyl-6-nitroaniline correspondante II

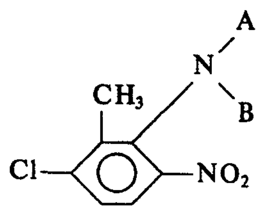

(II)

avec un phénol ou respectivement un thiophénol III

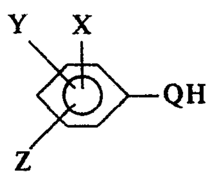

(III)

sous forme du phénolate ou respectivement du thiophénolate ou en présence d'un agent fixant les acides
ou en ce que
b) on fait réagir un 3-méthyl-2,4-diphénoxy-nitrobenzène correspondant IV

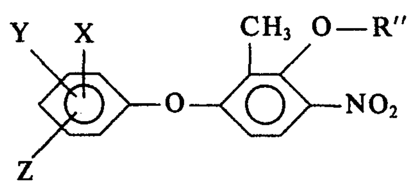

(IV)

dans lequel R'' remplace un radical phényle éventuellement substitué, de préférence le radical

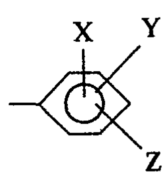

avec une amine V,

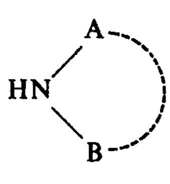

(V)

et en ce qu'éventuellement pour la préparation de composés de formule I dans lesquels B représente un groupe acyle, on introduit selon des méthodes usuelles un groupe acyle dans un composé correspondant avec B représentant hydrogène, ou en ce qu'éventuellement, pour la préparation de composés de formule I dans lesquels

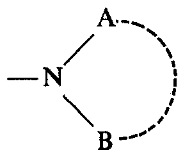

remplace $-N=CH-N(CH_3)_2$, on fait réagir un composé correspondant dans lequel A et B représentent l'hydrogène, avec un acétal de diméthylformamide ou avec du diméthylformamide/oxychlorure de phosphore.

10. Procédé pour la préparation de nouveaux agents herbicides, caractérisé en ce qu'on transforme un composé selon la revendication 1—4 au moyen d'adjuvants et/ou excipients usuels, en des concentrés ou respectivement agents prêts à l'emploi herbicides usuels.